# DEMANDE DE BREVET EUROPEEN

(11) **EP 1 230 912 A1**
(43) Date de publication de la demande: **14.08.2002**
(21) Numéro de dépôt: 02290086.4
(22) Date de dépôt: 14.01.2002
(51) Int. Cl.: A61K 7/48, A61K 7/40

(54) **Utilisation d'au moins un extrait de légumineuse comme agent anti-pollution, piégeur de métaux lourds.**

(30) Priorité: 07.02.2001 FR 0101642
(71) Demandeur: L'OREAL, 75008 Paris (FR)
(72) Inventeur: Pelletier, Pascale, 92160 Antony (FR); Catroux, Philippe, 94130 Nogent sur Marne (FR)
(74) Mandataire: Renard, Emmanuelle

(57) **Abrégé**

La présente demande se rapporte à l'utilisation en application topique d'au moins un extrait aqueux, alcoolique ou hydroalcoolique de légumineuse comme agent anti-pollution piégeur de métaux lourds, notamment comme agent cosmétique anti-pollution.

Un extrait de légumineuse utilisé est de préférence un extrait aqueux de maïs.

La demande se rapporte aussi à un procédé de traitement cosmétique en vue de protéger les matières kératiniques contre les effets de la pollution, en particulier contre les effets des métaux lourds, consistant à appliquer sur les matières kératiniques une composition contenant, dans un milieu physiologiquement acceptable, au moins un extrait de légumineuse tel que défini précédemment.

## Description

La présente demande se rapporte à l'utilisation en application topique, d'au moins un extrait de légumineuse comme agent anti-pollution piégeur de métaux lourds, et à un procédé de traitement cosmétique en vue de protéger les matières kératiniques contre les effets de la pollution, en particulier contre les effets des métaux lourds, comprenant l'application sur les matières kératiniques d'une composition contenant, dans un milieu physiologiquement acceptable, au moins un extrait aqueux, alcoolique ou hydroalcoolique de légumineuse.

Les milieux urbains sont régulièrement soumis à des pics de pollution. L'individu dans son environnement quotidien et particulièrement en zone urbaine, peut être soumis à de multiples agressions au niveau des matières kératiniques, et notamment de la peau, du cuir chevelu et des cheveux, par différents polluants aériens. Les polluants atmosphériques qui sont représentés largement par les produits primaires et secondaires de la combustion représentent une source importante du stress oxydatif environnemental. Différents types de produits chimiques, xénobiotiques et particules, composent la pollution urbaine. Trois grandes catégories de polluants peuvent exercer des effets délétères sur la peau et le cheveu : les gaz, les métaux lourds et les particules qui sont des résidus de combustion sur lesquelles sont absorbés de très nombreux composés organiques.

Ce sont les tissus les plus externes qui sont initialement et directement exposés aux toxiques de l'environnement. La peau est directement et fréquemment exposée à l'environnement pro-oxydant. Elle est particulièrement sensible à l'action du stress oxydatif, et sa couche la plus externe sert de barrière vis-à-vis des dommages oxydatifs. Dans la plupart des circonstances, l'oxydant a des chances d'être neutralisé après réaction avec les matières kératiniques, mais les produits de réaction formés peuvent être responsables d'atteintes cellulaires et tissulaires. Le stratum corneum, la barrière de la peau, est le site de contact entre air et tissu cutané. La structure biphasique lipides/protéines est un déterminant crucial de cette fonction de barrière de la peau. Ces éléments peuvent réagir avec les oxydants et être altérés ce qui favorisera les phénomènes de desquamation.

On sait par ailleurs que les métaux lourds sont des polluants atmosphériques dont les émissions ont notablement augmenté, notamment en milieu urbain ou industriel. Outre certains effets toxiques qui leur sont propres, les métaux lourds ont la propriété de diminuer l'activité des moyens de défense cellulaire contre les radicaux libres (J. Toxicol. Cut. & Ocular Toxical., 1987, 6(3), 183-191). Ainsi, les métaux lourds aggravent les effets toxiques des polluants gazeux en diminuant l'efficacité des moyens naturels de défense, et provoquent une accélération du phénomène de vieillissement cellulaire. Ceci est vrai en particulier pour les matières kératiniques et notamment la peau, le cuir chevelu et les cheveux qui sont en contact direct et permanent avec le milieu extérieur.

Parmi ces polluants aériens urbains de type métallique, on rencontre des métaux ou éléments métalloïdes comme le plomb, le cadmium, le mercure, l'arsenic (ou composés à base d'arsenic), le chrome (ou composés à base de chrome), des composés contenant du nickel, radon, berrylium, cadmium, silice, plomb...), (Environmental Health Perpectives, 1994,102:193-210). Ces éléments sont généralement adsorbés sur des particules de carbone en suspension dans l'air (notamment résidus de combustion des moteurs thermiques).

Il a été montré que certains métaux peuvent pénétrer dans la peau et s'accumuler (Critical Reviews in Toxicology, 1995, 25:397-462). La majorité des effets sont décrits dans des tissus tels que les poumons, les reins, le cerveau... A fortes concentrations ils peuvent induire :
- des mécanismes d'oxydation, en particulier des lipides membranaires,
- une cytotoxicité directe, capable d'aboutir à une nécrose cellulaire,
- une alkylation des nucléophiles cellulaires, mécanismes pouvant être à l'origine de phénomènes de sensibilisation ou de carcinogénèse,
(Free Radical Biology and Medicine, 1995, 18, 321-336.;The Journal of Biological Chemistry, 1998, 21, 12703-12709.; Biochemical and Molecular Medicine, 1995, 54, 33-37).

Ainsi, les effets nocifs de la pollution sur les matières kératiniques affectent la respiration cellulaire de ces matières kératiniques et se traduisent par un vieillissement accéléré de la peau, avec un teint terne et la formation précoce de rides ou ridules, et aussi par une diminution de la vigueur des cheveux qui prennent aussi un aspect terne. En outre, du fait de la pollution, peau et cheveux se salissent plus rapidement. De plus, la pollution peut provoquer des irritations et des phénomènes d'allergie et d'inflammation sur la peau.

Pour lutter contre ces effets des métaux lourds, divers agents anti-pollution ont été décrits. Ainsi le document EP-A-557 042 décrit l'utilisation des métallothionéines pour protéger les tissus contre les métaux lourds.

Avec l'augmentation de la pollution, il subsiste le besoin de trouver d'autres agents permettant de lutter efficacement contre l'effet néfaste des polluants sur les matières kératiniques et empêcher l'adhérence de ces polluants sur les matières kératiniques, notamment pour éviter la dégradation de la respiration cellulaire, la desquamation et le vieillissement accéléré des matières kératiniques et notamment de la peau, ainsi que lutter contre le teint terne et la formation précoce de rides ou ridules sur la peau, pour éviter que les cheveux aient un aspect terne et se salissent, et pour éviter l'irritation de la peau ainsi que les phénomènes d'allergie cutanée et d'inflammation de la peau.

La demanderesse a maintenant trouvé, de façon tout à fait surprenante, que l'utilisation d'un extrait aqueux, alcoolique ou hydroalcoolique de légumineuse, permettait de protéger les matières kératiniques des effets des polluants.

Ainsi, l'invention a pour objet l'utilisation cosmétique d'au moins un extrait aqueux, alcoolique ou hydroalcoolique de légumineuse, comme agent anti-pollution, dans une composition pour application topique sur les matières kératiniques.

Plus particulièrement, l'invention a pour objet l'utilisation cosmétique d'au moins un extrait aqueux, alcoolique ou hydroalcoolique de légumineuse, comme agent piégeur de métaux lourds, dans une composition pour application topique sur les matières kératiniques.

La présente invention concerne également l'utilisation d'au moins un extrait aqueux, alcoolique ou hydroalcoolique de légumineuse, pour la préparation d'une composition à application topique destinée à protéger les matières kératiniques contre les effets nocifs de la pollution.

Plus particulièrement la présente invention concerne l'utilisation d'au moins un extrait aqueux, alcoolique ou hydroalcoolique de légumineuse, pour la préparation d'une composition à application topique destinée à protéger les matières kératiniques contre les effets nocifs des métaux lourds.

Par extrait de légumineuse selon l'invention, on entend un extrait végétal de tubercule comme par exemple la pomme de terre, ou bien un extrait végétal de céréales comme par exemple le blé, le maïs, l'orge, ou le riz.

Les extraits de légumineuse utilisés dans le cadre de la présente invention peuvent être des extraits aqueux, alcooliques dont le solvant est constitué d'un ou plusieurs mono-alcools (par exemple l'éthanol) et/ou polyols, ou hydroalcooliques dont le solvant est constitué d'un mélange d'eau et de un ou plusieurs mono-alcools et/ou polyols (tels que glycol).

De préférence l'extrait de légumineuse est un extrait aqueuxe.

Pour la mise en oeuvre de l'invention les légumineuses seront avantageusement choisies parmi les céréales telles que le maïs, le blé, l'orge, ou le riz. Parmi ces céréales on choisira plus particulièrement le maïs.

Un extrait de maïs convenant particulièrement bien à la mise en oeuvre de la présente invention est l'extrait aqueux de maïs commercialisé par la société SOLABIA sous le nom de Phytovityl C. Cet extrait de maïs contient une matière active comprenant des protéines, des acides aminés (principalement acide glutamique, proline et alanine), du myo-inositol, des sels minéraux et de l'acide sorbique, dans un milieu comprenant de l'eau et du sorbitol.

La composition selon l'invention peut comprendre un ou plusieurs extraits de légumineuses.
On entend par « agent piégeur de métaux lourds » un agent qui agit en complexant et/ou chélatant les métaux lourds de façon à protèger la peau et les matières kératiniques et prévenir, atténuer et/ou supprimer les effets délétères de ces métaux.

Dans le cadre de la présente invention, on entend par « matière kératinique » la peau, le cuir chevelu, les cheveux, les cils, les sourcils, les ongles et les muqueuses.

On entend ici par « application topique » une application externe sur les matières kératiniques, que sont notamment la peau, le cuir chevelu, les cils, les sourcils, les ongles et les muqueuses.

L'extrait de légumineuse utilisé comme agent piégeur de métaux lourds selon l'invention est avantageusement en une quantité suffisante. On entend ici par « quantité suffisante » (ou quantité efficace), une quantité telle que la protection contre les polluants soit assurée. Cette quantité peut aller par exemple de 0,01 à 10 % en poids et de préférence de 0,05 à 5 % en poids de matière active de composé anti-pollution par rapport au poids total de la composition.

Les compositions à application topique, et notamment cosmétiques, utilisées selon l'invention contiennent un milieu physiologiquement acceptable, c'est-à-dire un milieu compatible avec la peau, les lèvres, le cuir chevelu, les cils, les yeux, les ongles et/ou les cheveux. Ce milieu physiologiquement acceptable peut être plus particulièrement constitué d'eau et éventuellement d'un solvant organique physiologiquement acceptable choisi par exemple parmi les alcools inférieurs comportant de 1 à 8 atomes de carbone et en particulier 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol. Ce peut être aussi un milieu anhydre, notamment un milieu huileux contenant des huiles et/ou matières grasses autres que les huiles.

Quand le milieu physiologiquement acceptable est un milieu aqueux, il a un pH compatible avec la peau, allant de préférence de 3 à 8 et mieux de 4 à 7.

Quand la composition comporte un milieu aqueux ou hydroalcoolique, il est possible d'ajouter une phase grasse (ou huileuse) dans ce milieu, afin que les compositions de l'invention soient plus douces et plus nourrissantes.

Ainsi, les compositions selon l'invention contenant les agents anti-pollution tels que définis ci-dessus peuvent se présenter sous toutes les formes galéniques classiquement utilisées pour une application topique et notamment sous forme de solutions aqueuses, hydroalcooliques ou huileuses, d'émulsions huile-dans-eau (H/E) ou eau-dans-huile (E/H) ou multiple (triple : E/H/E ou H/E/H), de gels aqueux ou huileux, de produits anhydres liquides, pâteux ou solides, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :.
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition contenant les composés anti-pollution est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de méthyl glucose commercialisé par Amerchol sous la dénomination deglutamate SSE 20, le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants hydrophiles ou lipophiles, les actifs hydrophiles ou lipophiles, les conservateurs, les antioxydants, les solvants, les parfums, les charges, les filtres UV, les bactéricides, les absorbeurs d'odeur, les matières colorantes, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Selon un mode préféré de réalisation de l'invention, la composition utilisée selon l'invention contient au moins un filtre UV (ou filtre solaire) qui peut être un filtre chimique ou un filtre physique ou un mélange de tels filtres.
A titre d'illustration et de façon non limitative, on peut citer les familles suivantes (les noms correspondent à la nomenclature CTFA des filtres) :
les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène di-camphre sulfonique , et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.); les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane, l'oxyde de zinc et le dioxyde de titane.

Des exemples de filtres UV convenant particulièrement bien à une utilisation dans la présente invention sont :
- le butyl méthoxydibenzoylméthane vendu notamment par la société HOFFMANN-LAROCHE sous la dénomination Parsol 1789,
- l'octocrylène vendu notamment par la société BASF sous la dénomination Uvinul N539,
- l'octyl salicylate vendu notamment par la société HAARMAN-REIMER sous la dénomination Neo Heliopan OS,
- l'octyl méthoxycinnamate vendu notamment par la société HOFFMANN-LAROCHE sous la dénomination Parsol MCX,
- l'acide phénylbenzimidazole sulfonique vendu notamment par la société MERCK sous la dénomination Eusolex 232,
- les oxybenzones telles que les benzophénones-3, -4 ou -5,
- les silicones benzotriazoles et en particulier le drométrizole trisiloxane,
- l'acide téréphtalylidène di-camphre sulfonique, et
- les oxydes de titane ou de zinc, sous forme de micro- ou nanoparticules éventuellement enrobées.

On utilise de préférence comme filtre dans la composition de l'invention la benzophénone-3, l'acide téréphtalylidène di-camphre sulfonique, l'octyl méthoxycinnamate, l'acide phénylbenzimidazole sulfonique, le drometrizole trisiloxane, le 4-methylbenzylidène camphre, l'anizotriazine, l'octocrylène, le butylmethoxydibenzoyl methane, l'oxyde de zinc et/ou le dioxyde de titane.

La quantité de filtres dépend de l'utilisation finale souhaitée. Elle peut aller par exemple de 1 à 20% en poids et mieux de 2 à 10% en poids par rapport au poids total de la composition.

Selon un autre mode préféré de réalisation selon l'invention, la composition utilisée contient en outre un actif antioxydant (par exemple la vitamine E).

Les compositions utilisées selon l'invention peuvent notamment constituer un produit de soin et/ou de maquillage des matières kératiniques, et notamment de la peau. Elles peuvent être utilisées notamment pour protéger les matières kératiniques, contre les effets de la pollution et plus particulièrement des métaux lourds, notamment pour améliorer la respiration cellulaire et/ou diminuer la desquamation et/ou pour éviter de ternir ou de salir les matières kératiniques, et notamment la peau.

Ainsi, un autre objet de l'invention consiste en un procédé de traitement cosmétique en vue de protéger les matières kératiniques contre les effets de la pollution et en particulier contre les effets des métaux lourds, comprenant l'application sur les matières kératiniques d'une composition contenant au moins un extrait aqueux, alcoolique ou hydroalcoolique de légumineuse.

L'invention a aussi pour objet un procédé de traitement cosmétique des matières kératiniques en vue d'améliorer leur respiration cellulaire et/ou de diminuer leur desquamation et/ou d'éviter de les ternir et/ou de les salir, comprenant l'application sur les matières kératiniques d'une composition contenant dans un milieu physiologiquement acceptable, au moins un extrait aqueux, alcoolique ou hydroalcoolique de légumineuse.

Les procédés de traitement cosmétique précédents seront avantageusement mis en oeuvre en utilisant un extrait de céréales et plus particulièrement un extrait de maïs.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

### Exemple 1 : Mise en évidence de l'effet protecteur d'un extrait de maïs sur des kératinocytes en culture vis à vis d'un métal lourd représentatif : le Cadmium.

### • PROTOCOLE EXPERIMENTAL

### Principe:

Les métaux lourds tels que le Cadmium, le Nickel, le Plomb, le Mercure etc... peuvent entraîner une cytotoxicité sur les cellules de différents organes dont la peau. L'effet cytotoxique du Cadmium sur kératinocytes humains en culture a été évalué par une technique de mesure de la viabilité cellulaire : test d'incorporation du Rouge Neutre (Culture Methods.1984, 9, 7-9). Cette méthode permet de mettre en évidence un effet protecteur de la molécule.

### Ensemencement des cellules:

L'étude a été réalisée sur une culture monocouche de kératinocytes humains immortalisés (DK7-NR) par le centre de recherches de Nestlé. Les cellules sont ensemencées à J-3 en boîtes de culture 96 puits à raison de 25000 cellules/cm² dans 100µl de milieu de culture (milieu défini sans sérum de veau, NR2, Biofluids). Les incubations sont réalisées en étuve à 37°C en atmosphère humide et enrichie à 5 % de CO2.

### Traitement par le polluant en présence du protecteur:

Les cellules sont traitées 24 heures par des concentrations croissantes (0, 10, 25, 50, 75, 100, 150 et 200 µM) de chlorure de cadmium (CdCl₂) seul, de façon à déterminer sa cytotoxicité. Parallèlement on réalise un traitement dans les mêmes conditions, mais en présence de l'extrait de maïs (Phytovityl® à 0.5 et 1%, concentrations non cytotoxiques pour les cellules). Les incubations sont réalisées en étuve à 37°C, 5% CO2 en atmosphère humide.

### Mesure de la viabilité cellulaire:

A la fin de la période de contact, la viabilité cellulaire est réalisée à l'aide d'un test d'incorporation de Rouge Neutre (POS 55/006), lecture spectrophotométrique à 550nm. Brièvement:
- Rincer les cellules par du tampon PBS afin d'éliminer les solutions de traitement
- ajouter 100µl par puits d'une solution à 0.5 mg/ml de rouge neutre dans le milieu de culture
- Incuber à 37°C, 5% CO2, atmosphère humide pendant 3 heures.
- Rincer au PBS
- Fixer avec une solution de formol/Calcium pendant 1 minute
- Extraire le rouge neutre par 100µl /puits d'une solution d'éthanol -acide acétique
- Lire la Densité Optique au spectrophotomètre à 550 nm.
- Calculer la concentration de CdCl2 entraînant une chute de 50% de la viabilité = CI-50

### • RESULTATS

Une étude préalable de cytotoxicité sur kératinocytes DK7-NR a été réalisée (test d'incorporation du rouge neutre). Après 24 heures de contact, la concentration maximale non cytotoxique de l'extrait de maïs est de 1 %. Son effet protecteur a été évalué à des concentrations égales ou inférieures à 1 % (P/V).

Les résultats obtenus montrent que l'extrait de maïs exerce un effet protecteur significatif vis à vis de la cytoxicité du Cadmium. Cet effet protecteur est clairement dose-dépendant comme le montre le tableau ci-dessous :

| **Concentration en extrait de maïs (%)** | **CI-50 (µM)** |
|---|---|
| 0 | 37,7 |
| 0,5 | 89,8 |
| 1 | 139,2 |

### Exemples de composition

### Exemple 2 : Emulsion H/E

| **Phase A (grasse) :** | |
|---|---|
| Monodiglycérylstéarate | 3 g |
| Huile de vaseline | 3 g |
| Alcool cétylique | 5 g |
| | |

| **Phase B (Phase aqueuse) :** | |
|---|---|
| Polyéthylène glycol oxyéthyléné par 50 moles d'oxyde d'éthylène | 3 g |
| Eau qsp | 100 g |
| | |

| **Phase C** | |
|---|---|
| Extrait de maïs (Phytovityl®) | 5 g |
| Eau | 10 g |

### Mode opératoire :

La phase grasse (A) et la phase aqueuse (B) sont préparées séparément et chauffées à 70°C. La phase grasse est versée dans la phase aqueuse sous agitation. L'émulsification est prolongée pendant 10 minutes, puis on refroidit lentement en agitant à une température de 40°. La phase C est ajoutée et on poursuit le refroidissement. On obtient une crème apte à être appliquée sur la peau pour la protéger contre les effets de la pollution.

### Exemple 3 :

De façon classique on formule la composition suivante :

| | |
|---|---|
| Extrait de maïs (Phytovityl®) | 1 g |
| Octylpalmitate | 10 g |
| glycérylisostéarate | 4 g |
| Huile de vaseline | 20 g |
| Sorbitol | 2 g |
| Vitamine E | 1 g |
| Glycérol | 3 g |
| Eau qsp | 100 g |

### Exemple 4 :

De façon classique on formule la composition suivante :

| | |
|---|---|
| Extrait de maïs (Phytovityl®) | 1 g |
| Octylpalmitate | 10 g |
| glycérylisostéarate | 4 g |
| Huile de purcellin | 23 g |
| Vitamine E | 1 g |
| Glycérol | 3 g |
| Eau qsp | 100 g |

### Exemple 5 : Gel

| **Phase A :** | |
|---|---|
| Eau | 10 g |
| Extrait de maïs (Phytovityl®) | 5 g |
| | |

| **Phase B :** | |
|---|---|
| Hydroxypropyle cellulose | 0.10 g |
| Carbopol ULTREZ 10 | 0.25 g |
| Polyéthylène glycol oxyéthyléné par 50 moles d'oxyde d'éthylène | 3 g |
| Eau qsp | 100 g |
| | |

| **Phase C** | |
|---|---|
| Triéthanolamine qsp pH 7 | |
| | |

| **Phase D** | |
|---|---|
| Timiron (Mica recouvert de titane) | 0.5% |

### Mode opératoire :

Les gélifiants de la phase B sont dispersés dans la phase A sous agitation vive. Le mélange obtenu est neutralisé avec la phase C. Enfin la phase D est dispersée sous agitation lente. On obtient un gel apte à être appliqué sur la peau pour la protéger contre les effets de la pollution.

## Revendications

1. Utilisation cosmétique d'au moins un extrait aqueux, alcoolique ou hydroalcoolique de légumineuse, comme agent anti-pollution, dans une composition pour application topique sur les matières kératiniques.

2. Utilisation cosmétique d'au moins un extrait aqueux, alcoolique ou hydroalcoolique de légumineuse, comme agent piégeur de métaux lourds, dans une composition pour application topique sur les matières kératiniques.

3. Utilisation d'au moins un extrait aqueux, alcoolique ou hydroalcoolique de légumineuse, pour la préparation d'une composition à application topique destinée à protéger les matières kératiniques contre les effets nocifs de la pollution.

4. Utilisation d'au moins un extrait aqueux, alcoolique ou hydroalcoolique de légumineuse, pour la préparation d'une composition à application topique destinée à protéger les matières kératiniques contre les effets nocifs des métaux lourds.

5. Utilisation selon la revendication 1 à 4, **caractérisée par le fait que** l'extrait de légumineuse est un extrait de céréales.

6. Utilisation selon l'une quelconque des revendications précédentes, **caractérisée par le fait que** l'extrait de légumineuse est un extrait aqueux.

7. Utilisation selon l'une quelconque des revendications précédentes, **caractérisée par le fait que** l'extrait de légumineuse est un extrait de maïs.

8. Utilisation selon l'une quelconque des revendications précédentes, **caractérisée par le fait que** la quantité d'extrait de légumineuse va de 0,01 à 10 % en poids par rapport au poids total de la composition.

9. Utilisation selon la revendication 8, **caractérisée par le fait que** la quantité d'extrait de légumineuse va de 0,05 à 5 % en poids par rapport au poids total de la composition.

10. Utilisation selon l'une quelconque des revendications 7 à 9, **caractérisée par le fait que** l'extrait de maïs contient un mélange de protéines, d'acides aminés, de myo-inositol, de sels minéraux et d'acide sorbique.

11. Utilisation selon l'une quelconque des revendications précédentes, **caractérisée en ce que** la composition contient en outre au moins un actif antioxydant.

12. Utilisation selon l'une quelconque des revendications précédentes, **caractérisée en ce que** la composition contient en outre au moins un filtre UV.

13. Procédé de traitement cosmétique en vue de protéger les matières kératiniques contre les effets de la pollution, comprenant l'application sur les matières kératiniques d'une composition contenant dans un milieu physiologiquement acceptable, au moins un extrait aqueux, alcoolique ou hydroalcoolique de légumineuse.

14. Procédé de traitement cosmétique en vue de protéger les matières kératiniques contre les effets des métaux lourds, comprenant l'application sur les matières kératiniques d'une composition contenant dans un milieu physiologiquement acceptable, au moins un extrait aqueux, alcoolique ou hydroalcoolique de légumineuse.

15. Procédé de traitement cosmétique des matières kératiniques en vue d'améliorer leur respiration cellulaire et/ou de diminuer leur desquamation et/ou d'éviter de les ternir et/ou de les salir, comprenant l'application sur les matières kératiniques d'une composition contenant, dans un milieu physiologiquement acceptable, au moins un extrait aqueux, alcoolique ou hydroalcoolique de légumineuse.

16. Procédé selon la revendication 13 à 15, **caractérisé par le fait que** l'extrait de légumineuse est un extrait aqueux de maïs.

17. Procédé selon l'une quelconque des revendications 13 à 16, **caractérisé par le fait que** la composition contient en outre au moins filtre UV.

18. Procédé selon l'une quelconque des revendications 13 à 17, **caractérisé par le fait que** la matière kératinique est la peau.
